## Europäisches Patentamt

## European Patent Office

## Office européen des brevets

(19)

(11) Veröffentlichungsnummer: **0 157 311**
**B1**

(12) # EUROPÄISCHE PATENTSCHRIFT

(45) Veröffentlichungstag der Patentschrift:
25.01.89

(51) Int. Cl.⁴: **C 07 C 69/533, C 07 C 67/54**

(21) Anmeldenummer: **85103387.8**

(22) Anmeldetag: **22.03.85**

(54) **Verfahren zur Abtrennung von 4-Pentensäuremethylester aus solchen und 3-Pentensäuremethylester enthaltenden Gemischen.**

(30) Priorität: **03.04.84 DE 3412295**

(43) Veröffentlichungstag der Anmeldung:
**09.10.85 Patentblatt 85/41**

(45) Bekanntmachung des Hinweises auf die Patenterteilung:
**25.01.89 Patentblatt 89/4**

(84) Benannte Vertragsstaaten:
**BE CH DE FR GB IT LI NL**

(56) Entgegenhaltungen:
**EP-A-0 126 349**
**US-A-2 741 632**
**US-A-3 161 672**
**US-A-3 214 347**

**PATENT ABSTRACTS OF JAPAN, Band 6, Nr. 68 (C-100) 946 , 30. April 1982; & JP - A - 57 7443 (MITSUBISCHI GAS KAGAKU) 14.01.1982**

(73) Patentinhaber: **BASF Aktiengesellschaft, Carl-Bosch- Strasse 38, D-6700 Ludwigshafen (DE)**

(72) Erfinder: **Schneider, Heinz- Walter, Dr., Bruesseler Ring 43, D-6700 Ludwigshafen (DE)**
Erfinder: **Kummer, Rudolf, Dr., Kreuzstrasse 6, D-6710 Frankenthal (DE)**
Erfinder: **Zimmerling, Dieter, Bruesseler Ring 43, D-6700 Ludwigshafen (DE)**

**Beschreibung**

Bei der Umsetzung von Butadien mit Kohlenmonoxid und Methanol in Gegenwart von Kobaltcarbonyl-Katalysatoren erhält man im wesentlichen 3-Pentensäuremethylester. Für weitere Umsetzungen, z. B. zur Herstellung von -Formylvaleriansäuremethylester durch Hydroformylierung ist es jedoch vorteilhaft, von 4-Pentensäuremethylester auszugehen.

4-Pentensäuremethylester erhält man z. B. durch Isomerisierung von 3-Pentensäuremethylester in Gegenwart von Komplexen aus Rhodiumtriphenylphosphin und Zinnchlorid wie in Tetrahedron, Vol. 28, Seiten 5769 bis 5777 (1972) beschrieben wird. Bei der Isomerisierung des 3-Pentensäuremethyl-esters erhält man Gemische aus 4-Pentensäuremethylester, 3-cis- und 3-trans-Pentensäuremethylester und 2-cis und 2-trans-Pentensäuremethylester im Gleichgewicht. Obzwar 4-Pentensäuremethylester um 7°C niedriger siedet als 3-Pentensäuremethylester, bedarf es eines hohen technischen Aufwandes, wie Kolonnen mit 100 Trennstufen und eines Rücklaufverhältnisses von 50, um 4-Pentensäuremethylester vom 3-Pentensäuremethylester abzutrennen.

Es war deshalb die technische Aufgabe gestellt, die Abtrennung von 4-Pentensäuremethylester von 3-Pentensäuremethylester einfacher zu gestalten und insbesondere bei der Destillation mit weniger Trennstufen und einem niedrigeren Rücklaufverhältnis auszukommen.

Diese Aufgabe wird gelöst in einem Verfahren zur Abtrennung von 4-Pentensäuremethylester aus solchen und 3-Pentensäuremethylester enthaltenden Gemischen durch Destillation, wobei man je Gewichtsteil 4-Pentensäuremethylester 0,25 bis 0,4 Gew.-Teile Wasser zugibt und das Azeotrop aus 4-Pentensäuremethylester und Wasser abdestilliert.

Das neue Verfahren hat den Vorteil, daß es technisch wenig aufwendig ist und es weniger Trennstufen und eines niedrigeren Rücklaufverhältnisses bedarf, um die isomeren Ester zu trennen.

Das neue Verfahren ist insofern bemerkenswert, als das Azeotrop 4-Pentensäuremethylester/Wasser bei 91,5°C siedet, während das Azeotrop aus 3-Pentensäuremethylester/Wasser bei 93,5°C siedet und somit lediglich eine Siedepunktsdifferenz von 2°C vorliegt, während die Siedepunktdifferenz für die Ester selbst 7°G beträgt.

Erfindungsgemäß geht man von Gemischen aus 4-Pentensäuremethylester und 3-Pentensäuremethylester aus. Diese Gemische enthalten in der Rgel außerdem 2-Pentensäuremethylester. Geeignete Gemische enthalten beispielsweise 3 bis 15 Gew.-% 4-Pentensäuremethylester, 75 bis 95 Gew.-% 3-Pentensäuremethylester und 0 bis 10 Gew.-% 2-Pentensäuremethylester. Geeignete Gemische erhält man beispielsweise durch Isomerisierung von 3-Pentensäuremethylester oder 2-Pentensäuremethylester bei erhöhter Temperatur in Gegenwart von sauren Ionenaustauschern oder sauren Zeolithen, die einen Gehalt an Edelmetallen der achten Gruppe des Periodischen Systems haben. Als Katalysatoren verwendet man bevorzugt Styroldivinylbenzolcopolymerisate mit Sulfonsäuregruppen oder saure Zeolithe vom A-, X- oder Y-Typ in der H-Form, d. h. in der sauren Form. Die sauren Ionenaustauscher oder sauren Zeolithe haben einen Gehalt vorzugsweise an Palladium, Ruthenium oder Rhodium, insbesondere Palladium von 0,01 bis 1 Gew.-%. Die Isomerisierung wird in der Regel bei einer Temperatur von 100 bis 150°C durchgeführt. Bei der Isomerisierung von 3-Pentensäuremethylester erhält man so bei einer Isomerisierungstemperatur von 135°C beispielsweise ein Isomerengemisch aus 8 Gew.-% 4-Pentensäuremethylester, 91 Gew.-% 3-Pentensäuremethylester und 0,5 Gew.-% 2-Pentensäuremethylester.

Der in dem Gemisch enthaltene 4-Pentensäuremethylester wird aus dem Gemisch durch Destillation abgetrennt, wobei man je Gewichtsteil 4-Penten-säuremethylester 0,25 bis 0,40 Gew.-Teile Wasser zugibt und das Azeotrop aus 4-Pentensäuremethylester und Wasser abdestilliert. Die Mengen des zugeführten Wassers richtet sich somit nach der Menge an 4-Pentensäuremethylester, die im Ausgangsgemisch enthalten ist und die für die Bildung des Azeotrops von 4-Pentensäuremethylester/Wasser mit einem Wasseranteil von 28,6 Gew.-% notwendig ist. Die erforderliche Wassermenge kann von Anfang an zugesetzt werden, es ist jedoch vorteilhaft, das Wasser in erforderlichem Maße während der Destillation fortwährend zuzugeben, z. B. in dem Maße, wie das Azeotrop 4-Pentensäuremethylester/Wasser am Kopf der Kolonne entnommen wird. Vorzugsweise wendet man höchstens die dem Azeotrop aus 4-Pentensäuremethylester und Wasser entsprechende Menge an Wasser an.

Die Destillation wird in der Regel in den üblichen Kolonnen, wie Füllkörperkolonnen, Glockenbödenkolonnen oder Siebbödenkolonnen, durchgeführt. Die Kolonnen haben vorteilhaft 20 bis 60 praktische Böden. Bei der Destillation hat es sich bewährt, wenn man ein Rückflußverhältnis von 1 : 2 bis 1 : 15 einhält. Durch die beschränkte Zugabe an Wasser, die nur zur Bildung des Azeotrops 4-Pentensäuremethylester/Wasser ausreicht, gelingt es, dieses Azeotrop selektiv von den restlichen Pentensäuremethylestern zu trennen. Aus dem anfallenden Destillat wird 4-Pentensäuremethylester durch Trennung in eine 4-Pentensäuremethylester-Phase und eine Wasser-Phase und nachfolgendes Abdekantieren gewonnen.

Die erfindungsgemäße Arbeitsweise läßt sich absatzweise durchführen, wird jedoch vorteilhaft kontinuierlich gestaltet.

4-Pentensäuremethylester eignen sich zur Herstellung von δ-Formylvaleriansäureestern, einem Vorprodukt für die Herstellung von Caprolactam.

Das Verfahren nach der Erfindung sei an folgenden Beispielen veranschaulicht.

**Beispiel 1**

In eine Kolonne von 80 cm Länge und 3 cm Durchmesser, die mit 3 mm Drahtwendeln gefüllt ist, werden stündlich 225 ml eines Gemisches aus 8 Gew.-% 4-Pentensäuremethylester, 91,7 Gew.-% 3-Pentensäuremethylester und 0,3 Gew.-% 2-Pentensäuremethylester zugeführt. Gleichzeitig werden am Kopf der Kolonne stündlich 6 ml Wasser zugepumpt. Die Temperatur im Sumpf der Kolonne wird bei 135 bis 137°C gehalten. Bei einem Rücklaufverhältnis von 1 : 10 erhält man stündlich 26 ml eines Gemisches aus Pentensäuremethylester und Wasser, das bei der Phasentrennung in 6 ml Wasser und 20 ml Pentensäureester zerfällt. Entsprechend gaschromatographischer Analyse erhält man 90 Gew.-% 4-Pentensäuremethylester und 10 Gew.-% 3-Pentensäuremethylester.

**Vergleichsbeispiel**

Man verfährt wie im Beispiel 1 beschrieben, führt jedoch kein Wasser zu. Bei einem Rücklaufverhältnis von 1 : 10 erhält man ein Pentensäuremethylestergemisch mit 47 Gew.-% 4-Pentensäuremethylester am Kopf der Kolonne.

**Patentansprüche**

1. Verfahren zur Abtrennung von 4-Pentensäuremethylester aus solchen und 3-Pentensäuremethylester enthaltenden Gemischen durch Destillation, dadurch gekennzeichnet, daß man je Gewichtsteil 4-Pentensäuremethylester 0,25 bis 0,40 Gew.%-Teile Wasser zugibt und das Azeotrop aus 4-Pentensäuremethylester und Wasser abdestilliert.

2. Verfahren nach Anspruch 1, dadurch gekennzeichnet, daß man höchstens die dem Azeotrop aus 4-Pentensäuremethylester und Wasser entsprechende Menge an Wasser zugibt.

**Claims**

1. A process for the isolation of methyl 4-pentenoate from a mixture containing it and methyl 3-pentenoate by distillation wherein from 0.25 to 0.40 part by weight of water is added per part by weight of methyl 4-pentenoate, and the azeotrope of methyl 4-pentenoate and water is distilled off.

2. A process as claimed in claim 1, wherein the amount of water added does not exceed the amount appropriate for the azeotrope of methyl 4-pentenoate and water.

**Revendications**

1. Procédé pour séparer le 4-penténate de méthyle de mélanges le contenant ainsi que le 3-penténate de méthyle, par distillation, caractérisé par le fait qu'on ajoute, par partie en poids de 4-penténate de méthyl, 0,25 à 0,40 % parties en poids d'eau et qu'on sépare par distallation l'azétrope du 4-penténate de méthyle et de l'eau.

2. Procédé sélon la revendication 1, caractérisé par le fait qu'on ajoute une quantité d'eau au plus égale à celle correspondant à l'azétrope du 4-penténate de méthyle et de l'eau.